# FASCICULE DE BREVET EUROPEEN

(11) **EP 0 180 514 B1**
(45) Date de publication et mention de la délivrance du brevet: **17.03.1993**
(21) Numéro de dépôt: 85402049.2
(22) Date de dépôt: 22.10.1985
(51) Int. Cl.: G01N 33/49, G01N 11/14

(54) **Procédé et dispositif de mesure de caractéristiques rhéologiques d'un fluide, en particulier d'un fluide biologique tel que le sang**
Verfahren und Vorrichtung zur Messung der rheologischen Eigenschaften eines biologischen Fluidums wie Blut
Method and apparatus for measuring the rheological properties of a biological fluid such as blood

(30) Priorité: 30.10.1984 FR 8416603
(43) Date de publication de la demande: 07.05.1986
(73) Titulaire: BERTIN & CIE, F-78373 Plaisir Cédex (FR)
(72) Inventeur: Mattout, Richard, F-92210 Saint-Cloud (FR)
(74) Mandataire: Ramey, Daniel

(56) Documents cités:
- FR-A- 2 487 520
- US-A- 3 053 078
- US-A- 4 152 927
- US-A- 4 299 119
- THE REVIEW OF SCIENTIFIC INSTRUMENTS, vol. 42, no. 10, octobre 1971, New York, NY (US); M.G.HODGINS et al., pp. 1455-1457

## Description

L'invention concerne un procédé et un dispositif de mesure de caractéristiques rhéologiques d'un fluide, en particulier de la viscosité d'un fluide biologique tel que le sang.

On connaît de très nombreuses réalisations de rhéomètres, qui sont du type rotatif et qui comprennent un cylindre fixe et contenant le fluide, et un élément cylindro-conique plongé dans le fluide et auquel est appliqué un couple de rotation. A partir des mesures du couple de rotation appliqué à cet élément et de sa vitesse de rotation autour de son axe, on peut déterminer la contrainte de cisaillement à laquelle est soumis Ie fluide au contact de l'élément rotatif, et la vitesse de cisaillement du fluide à la paroi de cet élément, et donc déterminer la viscosité du fluide, qui est le coefficient de proportionnalité entre la contrainte et la vitesse de cisaillement, et déterminer également la loi de variation de la viscosité en fonction des variations de la contrainte de cisaillement appliquée au fluide.

De plus, le mesure de la force axiale permet d'accéder à d'autres caractéristiques rhéologiques (en viscoélasticité par exemple).
Dans certains cas et, en particulier, lorsque le fluide est du sang ou un fluide biologique, les vitesses de rotation de l'élément mobile doivent être extrêmement faibles, par exemple de l'ordre d'un tour en plusieurs minutes, de sorte qu'il est très difficile de mesurer les couples et les vitesses de rotation de l'élément mobile avec une précision suffisante, sauf à utiliser des appareils très sophistiqués et très coûteux, dont l'utilisation est réservée à des spécialistes de haut niveau. En effet, les mesures réalisées avec ces appareils sont très délicates et longues à exécuter, notamment du fait que l'élément rotatif n'est que partiellement plongé dans le fluide et que les forces de tension superficielles à la traversée de la surface libre du fluide par le cylindre rotatif faussent les mesures. De plus, dans le cas du sang, la coagulation forme une croûte en surface qui est traversée par l'élément rotatif et fausse également les mesures. Il arrive également que l'élément rotatif soit guidé en rotation autour de son axe par un dispositif mécanique, dont les contacts de frottement avec une partie de cet élément, aussi faibles soient-ils, faussent également les mesures.

On connaît également des viscosimètres dans lesquels l'élément mobile est maintenu complètement immergé dans le fluide, pour éviter certains des inconvénients précités. Le document "The review of Scientific Instruments" volume 42, n^{.}10, 1971, pages 1455 à 1457, décrit un tel viscosimètre dans lequel l'élément tournant est maintenu complètement immergé dans le fluide par un champ électromagnétique de sustentation et est entraîné en rotation autour de son axe par un champ électromagnétique tournant produit par une bobine coaxiale alimentée par une puissance électrique constante. La viscosité du fluide est déduite de la durée d'une vingtaine de tours du cylindre tournant, moyennant un étalonnage avec de l'eau pure par exemple. La mesure de la période de rotation de l'élément tournant est très longue pour être très précise et se heurte alors à d'autres difficultés, par exemple de décantation des fluides biologiques. En outre, une seule valeur de la viscosité du fluide est déterminée, ce qui ne permet pas d'obtenir la loi de variation de la viscosité en fonction des variations de la contrainte de cisaillement appliquée au fluide.

L'invention a pour objet un rhéomètre de haute précision, qui ne présente pas les inconvénients des appareils antérieurs et qui peut être utilisé de façon simple, rapide et sûre par du personnel à faible qualification technique.

Elle propose à cet effet un procédé de mesure des caractéristiques rhéologiques d'un fluide, en particulier de la viscosité d'un fluide biologique tel que le sang, consistant à plonger un cylindre dans le fluide contenu dans un tube, à maintenir le cylindre complètement immergé dans le fluide sans contact avec le tube au moyen d'un champ magnétique ou électromagnétique de sustentation, à appliquer un couple de rotation à ce cylindre au moyen d'un champ électromagnétique tournant produit par une bobine qui entoure coaxialement le tube et qui est alimentée par un courant électrique de fréquence non nulle, caractérisé en ce qu'il consiste à mesurer la vitesse instantanée de rotation du cylindre et l'intensité du courant électrique alimentant la bobine, à répéter ces mesures pour différentes valeurs de l'intensité du courant électrique et à en déduire une loi de variation de la viscosité du fluide en fonction de la contrainte de cisaillement appliquée à ce fluide par la rotation du cylindre.

C'est l'intensité du courant électrique alimentant la bobine qui détermine la valeur du couple de rotation appliqué au cylindre, ce qui permet de mesurer de façon simple et précise, après un étalonnage préliminaire, la valeur du couple de rotation par mesure de l'intensité du courant. La mesure instantanée de la vitesse de rotation du cylindre permet d'effectuer plusieurs mesures pour plusieurs valeurs du couple de rotation du cylindre et donc d'obtenir une courbe de viscosité.

De plus, les mesures du couple de rotation appliqué au cylindre et de sa vitesse de rotation ne sont pas faussées par la tension superficielle du fluide à la traversée de sa surface libre par le cylindre, par une modification de cette surface libre ou par les contacts de frottement du cylindre avec les parois du tube contenant le fluide ou avec un dispositif de guidage en rotation du cylindre.

Selon une autre caractéristique de l'invention, le champ de sustentation précité est créé au moyen de deux champs magnétiques ou électromagnétiques coaxiaux au cylindre et agissant sur les extrémités axiales de celui-ci.

Avantageusement, le tube contenant le fluide et le cylindre rotatif est lui-même placé dans une chambre à température constante, de façon à éviter que les mesures de la viscosité du fluide soient influencées par des variations de température du fluide.

Le cylindre rotatif peut être entraîné à des vitesses de rotation très faibles qui correspondent à des vitesses de cisaillement du fluide très faibles, de l'ordre de 10⁻² à 1s⁻¹.

Cette caractéristique est particulièrement intéressante lorsque le fluide dont il faut mesurer la viscosité est du sang.

L'invention propose également un dispositif de mesure de caractéristiques rhéologiques d'un fluide, telles que sa viscosité, en particulier par exécution du procédé qui vient d'être décrit, ce dispositif comprenant un cylindre plongé dans le fluide contenu dans un tube, des moyens produisant un champ magnétique ou électromagnétique de sustentation agissant sur le cylindre pour le maintenir complètement immergé dans le fluide sans contact avec le tube, une bobine entourant coaxialement le tube et des moyens d'alimentation électrique de la bobine pour produire un champ électromagnétique tournant entraînant le cylindre en rotation, caractérisé en ce qu'il comprend des moyens de réglage de l'intensité du courant électrique alimentant la bobine, des moyens de mesure de cette intensité et des moyens de mesure de la vitesse de rotation instantanée correspondante du cylindre.

Dans ce dispositif, les moyens produisant le champ de sustentation du cylindre sont agencés en regard des extrémités axiales du cylindre et agissent sur ces extrémités axiales, des moyens de réglage du champ de sustentation étant prévus.

On peut ainsi régler le champ de sustentation pour un fluide particulier, de façon à obtenir l'immersion complète du cylindre rotatif dans le fluide, tout en évitant les contacts de frottement du cylindre sur les parois du tube.

Le cylindre rotatif peut présenter une densité inférieure ou supérieure à celle du fluide, et est dans les deux cas maintenu immergé dans le fluide par le champ de sustentation.

La mesure du déplacement axial ou de la force axiale du cylindre rotatif permet d'accéder à d'autres caractéristiques rhéologiques du fluide (par exemple en viscoélasticité).

Selon une autre caractéristique de l'invention, les moyens de mesure de la vitesse de rotation du cylindre comprennent au moins un capteur de proximité, monté fixement en regard d'une partie du cylindre comportant des plages électriquement conductrices qui sont régulièrement réparties sur la périphérie du cylindre.

On peut ainsi mesurer de façon précise et rapide des vitesses de rotation extrêmement faibles du cylindre.

Le capteur de proximité est avantageusement du type capacitif et comprend par exemple n plages métalliques portées par une enveloppe cylindrique fixe entourant coaxialement le tube contenant le fluide et le cylindre rotatif, et en regard desquelles défile un nombre différent de plages métalliques portées par le cylindre rotatif.

On améliore ainsi la précision de la mesure de la vitesse instantanée de rotation du cylindre, tout en simplifiant la structure du capteur de mesure et en réduisant son encombrement.

De préférence, le dispositif selon l'invention est associé à des circuits programmés de commande et d'exploitation, qui sont propres à commander la production du champ électromagnétique tournant et du champ de sustentation du cylindre rotatif, à vérifier si la vitesse de rotation du cylindre est constante avant d'effectuer une mesure, à faire varier selon une loi prédéterminée l'intensité du courant électrique produisant le champ électromagnétique tournant, à relever les couples de valeurs mesurées d'intensité du courant et de vitesse de rotation du cylindre, et à les convertir, au moyen de courbes d'étalonnage, en couples de valeurs de contrainte de cisaillement et de vitesse de cisaillement du fluide.

On peut ainsi, en réduisant à un minimum les réglages préalables, effectuer une série de resures permettant de tracer la courbe de variation de la viscosité en fonction de la contrainte de cisaillement du fluide.

Un tel dispositif est à fonctionnement quasi-automatique et peut être utilisé sans difficulté par du personnel ayant une faible qualification technique.

Dans la description qui suit, faite à titre d'exemple, on se réfère aux dessins annexés, dans lesquels :
- la figure 1 est une vue schématique en coupe axiale partielle d'un dispositif selon l'invention;
- la figure 2 est une vue schématique en coupe transversale d'une partie de ce dispositif;
- la figure 3 est une vue schématique partielle en coupe selon la ligne III-III de la figure 2.

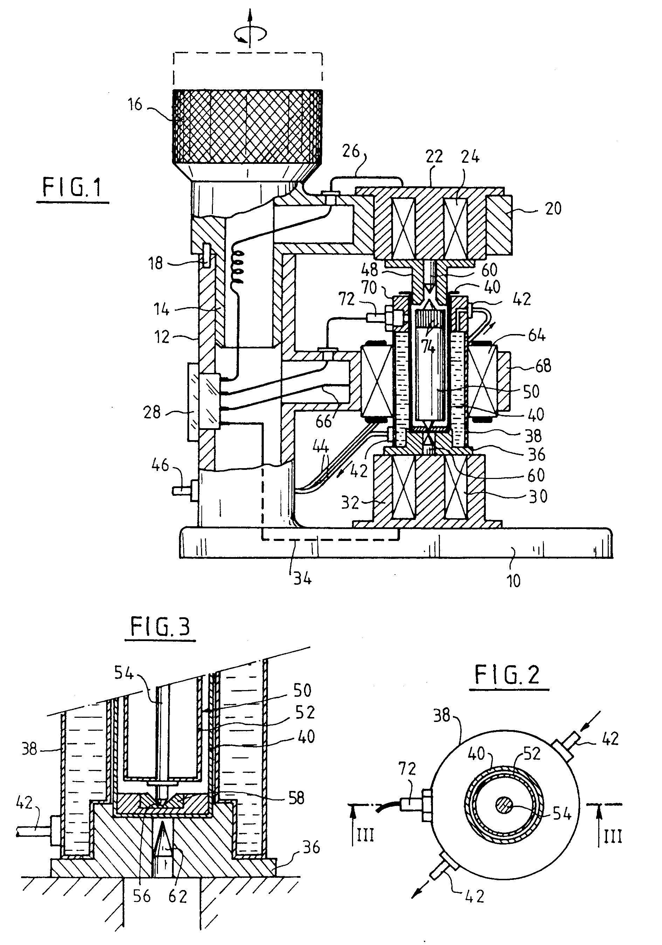

Le dispositif représenté en figure 1 comprend un pied 10 muni d'un montant vertical 12 tubulaire dans lequel s'engage le bras vertical tubulaire 14 d'une potence 16 qui peut être déplacée, par rapport au montant vertical 12,en translation le long d'un axe vertical et en rotation autour de cet axe, des moyens tels qu'une goupille ou un téton 18 étant prévus pour assurer un positionnement angulaire prédéterminé de la potence 16 par rapport au montant 12.

Le bras horizontal 20 de la potence 16 porte le noyau 22 d'une bobine d'induction 24 alimentée en courant électrique par des conducteurs 26 qui passent à l'intérieur des bras de la potence 16 et qui sont raccordés à une boîte de branchement 28 prévue sur le montant vertical 12.

Dans la position angulaire prédéterminée de la potence 16 par rapport au montant vertical 12 et au pied 10, la bobine 24 et son noyau 22 sont verticalement alignés avec une bobine 30 dont le noyau 32 est porté par le pied 10. La bobine 30 est alimentée en courant électrique par des conducteurs 34 aboutissant à la boîte de branchement 28 du montant vertical 12.

Sur la bobine 30 et son noyau 32 est placé un socle 36 de matière électriquement isolante et amagnétique, par exemple de matière plastique, qui supporte, à sa périphérie, l'extrémité inférieure d'une enceinte cylindrique tubulaire 38 et, dans sa partie centrale, l'extrémité inférieure d'un tube 40 à extrémité supérieure ouverte, qui est destiné à contenir le fluide dont il faut mesurer la viscosité.

L'enceinte tubulaire 38 est réalisée en matière amagnétique et électriquement isolante, par exemple en une matière plastique, et est parcourue en permanence par un courant de liquide, tel que de l'eau, à température constante. Pour cela, les extrémités supérieure et inférieure de l'enceinte 38 sont munies d'embouts 42 reliés par des conduits souples 44 à des moyens 46 de raccordement à un circuit de liquide à température constante, les moyens 46 étant prévus sur le montant vertical 12 du dispositif.

Le tube 40 est placé à l'intérieur de l'enceinte 38, sensiblement en contact avec la paroi interne de cette enceinte, et s'étend légèrement au-dessus de l'extrémité supérieure de l'enceinte 38. Ce tube 40 est réalisé en matière amagnétique et électriquement isolante, par exemple en matière plastique ou en verre, et son extrémité supérieure est maintenue par une pièce de centrage 48 en matière amagnétique et électriquement isolante, qui est fixée sous la bobine 24 et son noyau 22 portés par le bras horizontal 20 de la potence 16.

Le tube 40 contient, outre le fluide dont il faut mesurer la viscosité, un cylindre 50, disposé coaxialement à l'intérieur du tube 40, et qui est formé d'une enveloppe tubulaire cylindrique 52 en matière amagnétique et électriquement isolante, à extrméités fermées, et d'une tige cylindrique 54 de matière à propriétés magnétiques, par exemple de fer doux, qui s'étend sur toute la hauteur du cylindre 50 et traverse à étanchéité les extrémités fermées de l'enveloppe tubulaire 52.

L'extrémité inférieure de la tige 54 se termine par une bille 56 de matière très dure, par exemple d'iridium, qui est reçue dans une crapaudine 58 disposée sur le fond du tube 40.

Les extrémités de la tige 54 sont disposées en regard de pièces polaires 60 et 62 associées aux bobines 24 et 30 respectivement, et qui sont logées axialement dans les pièces de centrage 48 et 36 respectivement.

L'enceinte 38 est entourée coaxialement par une bobine d'induction 64, alimentée en courant alternatif par des conducteurs 66 aboutissant à la boîte de branchement 28. La bobine 64 est portée par un bras horizontal 68 solidaire du montant vertical 12.

La partie supérieure de l'enceinte 38 peut comprendre, comme représenté en figure 1, une bague cylindrique 70 de matière amagnétique et électriquement isolante,comprenant au moins un logement radial dans lequel est monté un capteur de proximité 72 raccordé également à la boîte de branchement 28. Ce capteur 72 détecte le défilement de plages métalliques électroconductrices 74 prévues à l'extrémité supérieure de l'enveloppe 52 du cylindre rotatif 50 et régulièrement réparties à la périphérie cylindrique de cette enveloppe.

Le capteur de proximité 72 est par exemple du type capacitif et peut être constitué lui-même par des séries de plages métalliques électroconductrices réparties régulièrement sur la périphérie interne de la bague 70. Dans ce cas, la bague 70 peut comprendre n plages métalliques, tandis que l'extrémité supérieure du cylindre rotatif 50 comprend par exemple n+1 ou n-1 plages conductrices. Lorsque les plages conductrices du cylindre rotatif sont reliées en parallèle à une bague conductrice du cylindre et que les plages conductrices de la bague 70 sont reliées en série entre elles et à une borne d'une source d'alimentation électrique dont l'autre borne est reliée à une piste conductrice circulaire entourant à distance la bague conductrice du cylindre rotatif, on obtient, à la rotation du cylindre, un signal sinusoïdal dont la fréquence correspond à la vitesse de rotation du cylindre rotatif.

Le dispositif qui vient d'être décrit est utilisé de la façon suivante :
On place à l'intérieur de l'enceinte 38 un tube 40 contenant le fluide, par exemple du sang ou un fluide biologique, dont il faut mesurer la viscosité. Le cylindre 50, dont la densité est par exemple supérieure à celle du fluide, est placé dans le tube et est complètement immergé dans le fluide. La bille 56 de l'extrémité inférieure de la tige 54 de ce cylindre est alors reçue dans la crapaudine 58 de l'extrémité inférieure du tube 40. La circulation d'un liquide à température constante dans l'enceinte 38 maintient la température du tube 40, du fluide et du cylindre 50 à une valeur constante.

La bobine 64 est alimentée en courant alternatif, par exemple par un générateur basse fréquence, comprenant des moyens de réglage de l'intensité du courant. La bobine 64 produit un champ électromagnétique tournant auquel est soumise la tige 54 de fer doux ou de matière analogue du cylindre rotatif 50. On applique ainsi à ce cylindre un couple de rotation autour de son axe vertical. L'intensité du courant d'alimentation de la bobine 64 détermine la valeur de ce couple de rotation, la fréquence de ce courant étant choisie à une valeur quelconque non nulle.

Les bobines 24 et 30 sont également alimentées en courant électrique et produisent, par leurs pièces polaires 60 et 62, un champ électromagnétique de sustentation auquel sont soumises les extrémités en pointe de la tige 54 du cylindre rotatif 50. Sous l'effet de ce champ électromagnétique, l'extrémité inférieure de la tige 54 s'écarte de la crapaudine 58, le cylindre 50 restant alors totalement immergé dans le fluide.

La vitesse de rotation du cylindre est mesurée au moyen du ou des capteurs de proximité 72. Lorsque cette vitesse de rotation devient constante, pour une intensité donnée du courant d'alimentation de la bobine 64, on relève les valeurs de la vitesse de rotation et de l'intensité du courant. Des courbes d'étalonnage permettent de déterminer, à partir de ces mesures, la valeur de la vitesse de cisaillement du fluide, et celle de sa contrainte de cisaillement. De ces deux valeurs, on tire la valeur de la viscosité du fluide.

Cette viscosité n'étant pas constante en fonction de la contrainte de cisaillement, on modifie la valeur de l'intensité du courant d'alimentation de la bobine 64, et l'on fait une nouvelle mesure de la vitesse de rotation du cylindre rotatif, pour obtenir une nouvelle valeur de la viscosité. On peut ainsi tracer point par point la courbe de variation de la viscosité en fonction des variations de la contrainte de cisaillement du fluide.

Pour fixer les idées, on précisera, à titre d'exemple, que le cylindre rotatif peut avoir un diamètre extérieur d'un centimètre environ, une longueur de trois à quatre centimètres,et un poids de trois à quatre grammes. L'espace annulaire cylindrique entre le cylindre rotatif et le tube 40 a une dimension radiale de l'ordre de 1 mm. On comprend que la quantité de fluide qui est placée dans le tube 40 est relativement très faible. La vitesse de rotation du cylindre 50 est de l'ordre de 1 tour par minute par exemple, ce qui correspond à des vitesses de cisaillement du fluide inférieures à 1s⁻¹.

De façon générale, le dispositif selon l'invention permet de mesurer des vitesses de cisaillement comprises entre 10³ et 10⁻² s⁻¹.

Lorsqu'une série de mesures est terminée pour un échantillon d'un fluide donné, il suffit de retirer le tube 40 de l'enceinte 38 et de le remplacer par un autre tube contenant un autre échantillon de fluide et un autre cylindre rotatif.

Avantageusement, le dispositif selon l'invention est associé à des circuits programmés de commande, qui permettent de réaliser automatiquement ou quasi-automatiquement les séquences décrites d'opérations et de mesures. Par exemple, ces circuits réalisent les fonctions suivantes :
- alimentation de la bobine 64 et des bobines 24 et 30, vérification de la sustentation du cylindre rotatif et de son immersion complète dans le fluide, vérification de la vitesse de rotation du cylindre,
- variation, selon une loi prédéterminée, de l'intensité du courant d'alimentation de la bobine 64 et relevé des couples de valeurs mesurées de l'intensité du courant et de la vitesse de rotation du cylindre,
- conversion des intensités de courant en contrainte de cisaillement et des vitesses de rotation en vitesses de cisaillement, au moyen de courbes d'étalonnage enregistrées en mémoire, et impression des résultats correspondants ou tracé d'une courbe de variation correspondante de la viscosité d'un fluide.

D'autres variantes et modifications peuvent être apportées au dispositif qui vient d'être décrit, sans sortir du cadre de l'invention. Par exemple, le cylindre rotatif peut avoir une densité inférieure à celle du fluide contenu dans le tube 40. Dans ce cas, ce cylindre rotatif aurait tendance à flotter sur le fluide, mais est maintenu immergé et en suspension dans le fluide par le champ électromagnétique produit par les bobines 24 et 30 et leurs pièces polaires 60 et 62.

Ce champ de sustentation peut être produit de façon magnétique et électromagnétique, par combinaison d'aimants permanents et de bobines d'induction, et peut être variable. Il peut également être produit,dans certains cas, uniquement par des aimants permanents.

Par ailleurs, le dispositif selon l'invention pourrait être à disposition horizontale, au lieu d'être à disposition verticale comme représenté en figure 1. Pour cela,les bobines 24 et 30 seraient alignées horizontalement, l'enceinte 38 serait d'axe horizontal et le tube 40 placé dans cette enceinte devrait être fermé hermétiquementà ses deux extrémités et totalement rempli de fluide.

D'autres moyens de mesure de la vitesse de rotation du capteur 50 peuvent également être utilisés, pour autant qu'ils soient suffisamment précis dans la mesure des vitesses faibles de rotation.

L'élément rotatif n'est pas nécessairement cylindrique et peut avoir une autre forme, par exemple cylindro-conique, selon les caractéristiques rhéologiques à mesurer.

De façon générale, le procédé et le dispositif selon l'invention permettent d'effectuer des mesures précises de la viscosité d'un fluide quelconque, avec une précision meilleure que 1% et pour un prix d'appareillage relativement faible.

## Revendications

1. Procédé de mesure des caractéristiques rhéologiques d'un fluide, en particulier de la viscosité d'un fluide biologique tel que le sang, consistant à plonger un cylindre (50) dans le fluide contenu dans un tube (40), à maintenir le cylindre (50) complètement immergé dans le fluide sans contact avec le tube (40) au moyen d'un champ magnétique ou électromagnétique de sustentation, à appliquer un couple de rotation au cylindre (50) au moyen d'un champ électromagnétique tournant produit par une bobine (64) qui entoure coaxialement le tube (40) et qui est alimentée par un courant électrique de fréquence non nulle, caractérisé en ce qu'il consiste à mesurer la vitesse instantanée de rotation du cylindre (50) et l'intensité du courant électrique alimentant la bobine (64), à répéter ces mesures pour différentes valeurs de l'intensité du courant électrique, et à en déduire une loi de variation de la viscosité du fluide en fonction de la contrainte de cisaillement appliquée à ce fluide par la rotation du cylindre.

2. Procédé selon la revendication 1, caractérisé en ce que le champ de sustentation précité est créé au moyen de deux champs magnétiques ou électromagnétiques coaxiaux au cylindre (50) et agissant sur les extrémités axiales de celui-ci.

3. Procédé selon la revendication 1 ou 2, caractérisé en ce qu'il consiste à placer le tube (40) contenant le fluide et le cylindre (50) dans une enceinte (38) à température constante.

4. Procédé selon l'une des revendications précédentes, caractérisé en ce qu'il consiste à entraîner le cylindre (50) en rotation à des vitesses très faibles, correspondant à des vitesses de cisaillement du fluide très faible, de l'ordre de 1 à 10⁻²s⁻¹.

5. Dispositif de mesure des caractéristiques rhéologiques d'un fluide, telles que sa viscosité, en particulier par exécution du procédé selon l'une des revendications 1 à 4, comprenant un cylindre (50) plongé dans le fluide contenu dans un tube (40), des moyens (24,30) produisant un champ magnétique ou électromagnétique de sustentation agissant sur le cylindre (50) pour le maintenir complètement immergé dans le fluide sans contact avec le tube (40), une bobine (64) entourant coaxialement le tube (40), et des moyens d'alimentation électrique de la bobine par un courant électrique de fréquence quelconque non nulle pour produire un champ électromagnétique tournant entraînant le cylindre (50) en rotation, caractérisé en ce qu'il comprend des moyens de réglage de l'intensité du courant électrique alimentant la bobine (64), des moyens de mesure de cette intensité et des moyens (72) de mesure de la vitesse de rotation instantanée correspondante du cylindre (50).

6. Dispositif selon la revendication 5, caractérisé en ce que les moyens produisant le champ de sustentation du cylindre (50) sont agencés en regard des extrémités axiales de ce cylindre et agissent sur ces extrémités axiales, des moyens de réglage du champ de sustentation étant également prévus.

7. Dispositif selon la revendication 5 ou 6, caractérisé en ce que le cylindre rotatif (50) a une densité inférieure à celle du fluide et est maintenu immergé dans le fluide par le champ de sustentation.

8. Dispositif selon la revendication 5 ou 6, caractérisé en ce que le cylindre (50) a une densité supérieure à celle du fluide et est maintenu par le champ de sustentation en suspension dans le fluide contenu dans le tube (40).

9. Dispositif selon l'une des revendications 5 à 8, caractérisé en ce que le cylindre rotatif (50) comprend une enveloppe cylindrique creuse (52) en matière amagnétique, fermée à ses extrémités, et une tige axiale (54) en matière magnétique, traversant à étanchéité les extrémités de l'enveloppe (52).

10. Dispositif selon la revendication 9, caractérisé en ce que la tige axiale (54) du cylindre se termine à son extrémité inférieure par une bille (56) propre à être reçue dans une crapaudine (58) de l'extrémité du tube (40), à l'arrêt du cylindre.

11. Dispositif selon l'une des revendications 5 à 10, caractérisé en ce que le tube (40) contenant le fluide et le cylindre (50) est placé dans une enceinte (30) parcourue par un courant de liquide, par exemple d'eau, à température constante.

12. Dispositif selon l'une des revendications 5 à 11, caractérisé en ce que les moyens de mesure de la vitesse de rotation instantanée du cylindre (50) comprennent au moins un capteur de proximité (72) monté fixement au niveau d'une partie du cylindre (50) comportant des plages (74) électriquement conductrices réparties régulièrement sur la périphérie du cylindre.

13. Dispositif selon la revendication 12, caractérisé en ce que le capteur de proximité (72) est du type capacitif et comprend par exemple n plages métalliques portées par une bague (70) cylindrique fixe entourant coaxialement le tube (40) et en regard desquelles défile un nombre différent de plages métalliques (74) portées par le cylindre rotatif (50).

14. Dispositif selon l'une des revendications 5 à 13, caractérisé en ce qu'il est associé à des circuits programmés de commande et d'exploitation, propres à commander la production du champ électromagnétique tournant et du champ de sustentation du cylindre rotatif (50), à vérifier si la vitesse de rotation du cylindre est constante avant d'effectuer une mesure, à faire varier selon une loi prédéterminée l'intensité du courant électrique produisant le champ électromagnétique tournant, à relever les couples de valeurs mesurées d'intensité du courant et de vitesse de rotation instantanée du cylindre, et à les convertir au moyen de courbes d'étalonnage, en couples de valeurs de contrainte de cisaillement et vitesse de cisaillement du fluide.

## Claims

1. A method of measuring the rheological characteristics of a fluid, in particular the viscosity of a biological fluid such as blood, the method consisting in dipping a cylinder (50) into the fluid contained in a tube (40), in keeping the cylinder (50) completely immersed in the fluid without making contact with the tube (40) by means of a magnetic or an electromagnetic support field, in applying a rotary torque to the cylinder (50) by means of a rotary electromagnetic field produced by a winding (64) which surrounds the tube (40) coaxially and which is fed with electrical current at a non-zero frequency, the method being characterized in that it consists in measuring the instantaneous speed of rotation of the cylinder (50) and the intensity of the electrical current, in repeating these measurements for different values of the intensity of the electrical current, and in deducing therefrom a relationship showing how the viscosity of the fluid varies as a function of the shear stress applied to said fluid by the rotation of the cylinder.

2. A method according to claim 1, characterized in that the above-mentioned support field is created by means of two magnetic or electromagnetic fields that are coaxial with the cylinder (50) and that act on the axial ends thereof.

3. A method according to claim 1 or 2, characterized in that it consists in placing the tube (40) containing the fluid and the cylinder (50) in a constant temperature jacket (38).

4. A method according to any preceding claim, characterized in that it consists in rotating the cylinder (50) at very slow speeds, corresponding to very low fluid shear speeds, of about 1 s⁻¹ to 10⁻² s⁻¹.

5. A device for measuring the rheological characteristics of a fluid, such as its viscosity, in particular by performing the method according to any one of claims 1 to 4, the device comprising a cylinder (50) dipped in the fluid contained in a tube (40), means (24, 30) producing a magnetic or an electromagnetic support field acting on the cylinder (50) for holding it completely immersed in the fluid without making contact with the tube (40), a winding (64) surrounding the tube (40) coaxially, and means for feeding the winding with an electrical current at an arbitrary non-zero frequency to produce a rotating electromagnetic field that rotates the cylinder (50), the device being characterized in that it includes means for adjusting the intensity of the electrical current fed to the winding (64), means for measuring said current intensity, and means (72) for measuring the corresponding instantaneous speed of rotation of the cylinder (50).

6. A device according to claim 5, characterized in that the means producing the cylinder supporting field are disposed facing the axial ends of the cylinder (50) and act on said axial ends, means also being provided for adjusting the supporting field.

7. A device according to claim 5 or 6, characterized in that the density of the rotary cylinder (50) is less than the density of the fluid and in that the cylinder is held immersed in the fluid by the support field.

8. A device according to claim 5 or 6, characterized in that the density of the cylinder (50) is greater than that of the fluid and in that the cylinder is held in suspension in the fluid contained in the tube (40) by the support field.

9. A device according to any one of claims 5 to 8, characterized in that the rotary cylinder (50) includes a hollow cylindrical envelope (52) of non-magnetic material that is closed at its ends, and an axial rod made of magnetic material that passes in sealed manner through the end of said envelope (52).

10. A device according to claim 9, characterized in that the axial rod (54) of the cylinder is terminated at its bottom end by a ball (56) suitable for being received in a cup (58) at the end of the tube (40), when the cylinder comes to rest.

11. A device according to any one of claims 5 to 10, characterized in that the tube (40) containing the fluid and the cylinder (50) is placed in a jacket (38) through which a flow of liquid, e.g. water, passes at constant temperature.

12. A device according to any one of claims 5 to 11, characterized in that the means for measuring the instantaneous speed of rotation of the cylinder (50) comprise at least one proximity sensor (72) fixed level with a portion of the cylinder (50) that includes electrically-conductive regions (74) which are regularly distributed around the periphery of the cylinder.

13. A device according to claim 12, characterized in that the proximity sensor (72) is of the capacitive type and comprises, for example, n metal regions mounted on a cylindrical ring (70) which coaxially surrounds the tube (40) with a different number of metal regions (74) mounted on the rotary cylinder (50) running past it.

14. A device according to any one of claims 5 to 13, characterized in that it is associated with programmed control and operation circuits suitable for controlling the production of the rotating electromagnetic field and for controlling the production of the cylinder-support field, for checking whether the speed of rotation of the cylinder (50) is constant prior to performing a measurement, for varying the intensity of the electric current producing the rotating electromagnetic field in accordance with a predetermined relationship, for storing pairs of measured values concerning the current intensity and the speed of rotation of the cylinder, and for converting said pairs of values by means of calibration curves into pairs of values concerning shear stress and shear speed in the fluid.

## Patentansprüche

1. Verfahren zum Messen rheologischer Eigenschaften eines Fluidums, insbesondere der Viskosität eines biologische Fluidum wie Blut, das folgendes aufweist: Eintauchen eines Zylinders (50) in das in einer Röhre (40) enthaltenen Fluidums, vollständig eingetaucht Halten des Zylinders (50) in dem Fluidum, ohne Berührung mit der Röhre (40) mittels eines magnetischen oder elektromagnetischen Schwebefeldes, Anwenden eines Drehmomentes auf den Zylinder (50) mittels eines elektromagnetischen Drehfeldes, das von einer Spule (64) erzeugt wird, welche die Röhre (40) koaxial umschließt und die mit elektrischem Strom gespeist wird, dessen Frequenz größer Null ist, **dadurch gekennzeichnet, daß** es folgendes aufweist: Messen der momentanen Rotationsgeschwindigkeit des Zylinders (50) und der Intensität des elektrischen Stromes, der die Spule (64) speist, Wiederholen dieser Messungen für unterschiedliche Werte der Intensität des elektrischen Stromes und Herleiten eines Gesetzes für die Änderung der Viskosität des Fluidums in Abhängigkeit von der durch die Rotation des Zylinders auf dieses Fluidum aufgebrachten Schubspannung.

2. Verfahren nach Anspruch 1, **dadurch gekennzeichnet, daß** das Schwebefeld mittels zweier magnetischer oder elektromagnetischer, bezüglich des Zylinders (50) koaxialer Felder erzeugt wird, die an seinen axialen Enden angreifen.

3. Verfahren nach Anspruch 1 oder 2, **dadurch gekennzeichnet, daß** die Röhre (40), die das Fluidum und den Zylinder (50) enthält, in einem Gefäß (38) mit konstanter Temperatur angeordnet wird.

4. Verfahren nach einem der vorigen Ansprüche, **dadurch gekennzeichnet, daß** der Zylinder (50) mit sehr geringen Rotationsgeschwindigkeiten angetrieben wird, welche sehr geringen Schubgeschwindigkeiten des Fluidums in der Größenordnung von 1 bis 10⁻² s⁻¹ entsprechen.

5. Vorrichtung zum Messen rheologischer Eigenschaften eines Fluidums, wie seine Viskosität, insbesondere durch Ausführen des Verfahrens nach einem der Ansprüche 1 bis 4, die aufweist: einen Zylinder (50), der in das in einer Röhre (40) enthaltene Fluidum eingetaucht ist, Mittel (24, 30) zum Herstellen eines magnetischen oder elektromagnetischen Schwebefeldes, das an dem Zylinder (50) angreift, um ihn in der Röhre (40) ohne Berührung der Röhre (40) vollständig eingetaucht zu halten, eine Spule (64), welche die Röhre (40) koaxial umschließt und Mittel zur Speisung der Spule mit einem elektrischen Strom, dessen Frequenz größer Null ist, um ein elektromagnetisches Drehfeld zu erzeugen, das den Zylinder (50) antreibt, **dadurch gekennzeichnet, daß** sie folgendes aufweist: Mittel zum Regeln der Intensität des elektrischen Stromes, welcher die Spule (64) speist, Mittel zum Messen dieser Intensität und Mittel zum Messen der momentanen, dem Zylinder (50) zugeordneten Rotationsgeschwindigkeit.

6. Vorrichtung nach Anspruch 5, **dadurch gekennzeichnet, daß** die Mittel zum Erzeugen des Feldes für das Schweben des Zylinders (50) bezüglich der axialen Enden dieses Zylinders angeordnet sind und an diesen axialen Enden wirken, wobei ebenso Mittel zum Regeln des Schwebefeldes vorgesehen sind.

7. Vorrichtung nach Anspruch 5 oder 6, **dadurch gekennzeichnet, daß** der rotierende Zylinder (50) eine geringere Dichte als das Fluidum besitzt und mittels des Schwebefeldes in dem Fluidum untergetaucht gehalten wird.

8. Vorrichtung nach Anspruch 5 oder 6, **dadurch gekennzeichnet, daß** der Zylinder (50) eine größere Dichte als das Fluidum besitzt und mittels des Schwebefeldes in der das Fluidum enthaltenden Röhre (40) in Schwebe gehalten wird.

9. Vorrichtung nach einem der Ansprüche 5 bis 8, **dadurch gekennzeichnet, daß** der rotierende Zylinder (50) eine hohle zylindrische Hülle (52) aus antimagnetischem Material, die an ihren Enden geschlossen ist, und eine axiale Welle (54) aus magnetischem Material aufweist, welche dicht durch die Enden der Hülle (52) hindurchreicht.

10. Vorrichtung nach Anspruch 9, **dadurch gekennzeichnet, daß** die axiale Welle (54) des Zylinders an ihrem unteren Ende in einer Kugel (56) endet, die bei Anhalten des Zylinders am Ende der Röhre (40) in einer Lagerpfanne (58) aufgenommen werden kann.

11. Vorrichtung nach einem der Ansprüche 5 bis 10, **dadurch gekennzeichnet, daß** die Röhre (40), welche das Fluidum und den Zylinder (50) enthält, in einem Gehäuse (38) angeordnet ist, das von einer Flüssigkeit, beispielsweise Wasser mit konstanter Temperatur durchströmt wird.

12. Vorrichtung nach einem der Ansprüche 5 bis 11, **dadurch gekennzeichnet, daß** die Mittel zum Messen der momentanen Rotationsgeschwindigkeit des Zylinders (50) zumindest einen berührungslosen Sensor (72) aufweisen, der an der Höhe des Zylinderteils (50) fest montiert ist, welcher elektrisch leitfähige Bereiche (74) aufweist, die gleichmäßig über den Umfang des Zylinders verteilt sind.

13. Vorrichtung nach Anspruch 12, **dadurch gekennzeichnet, daß** der berührungslose Sensor (72) kapazitiv wirkt und beispielsweise n metallische Bereiche aufweist, die an einem zylindrischen Ring (70) angeordnet sind, der koaxial rund um die Röhre (40) befestigt ist und an dem eine unterschiedliche Anzahl metallischer Platten (74) vorbeilaufen, die an dem rotierenden Zylinder (50) angebracht sind.

14. Vorrichtung nach einem der Ansprüche 5 bis 13, **dadurch gekennzeichnet, daß** sie programmierten Steuer- und Auswerteschaltungen zugeordnet ist, die für folgendes geeignet sind: zum Erzeugen des elektromagnetischen Drehfeldes und des Feldes, das den rotierenden Zylinder (50) in Schwebe hält, zum Verifizieren, ob die Rotationsgeschwindigkeit des Zylinders nach dem Ausführen einer Messung konstant ist, zum Verändern der Intensität des das elektromagnetische Drehfeld erzeugenden elektrischen Stromes gemäß eines vorbestimmten Gesetzes, zum nochmaligen Feststellen der Meßwertpaare von Intensität des Stromes und der momentanen Rotationsgeschwindigkeit des Zylinders und zu ihrem Umwandeln in Wertepaare von Schubspannung und Schubgeschwindigkeit des Fluidums mit Hilfe von Eichkurven.
